# EUROPEAN PATENT APPLICATION

(11) **EP 3 949 855 A1**
(43) Date of publication of application: **09.02.2022**
(21) Application number: 20189563.8
(22) Date of filing: 05.08.2020
(51) Int. Cl.: A61B 5/097, A61B 5/087

(54) **DISPOSABLE ANTIMICROBIAL FILTER FOR SPIROMETRY**

(71) Applicant: COSMED S.r.l., 00197 Roma (RM) (IT)
(72) Inventor: BRUGNOLI, Paolo, 00041 Pavona di Albano (Roma (IT); CHIORBOLI, Simone, 00041 Pavona di Albano (RM) (IT)
(74) Representative: Notaro, Giancarlo

(57) **Abstract**

A disposable antimicrobial filter (1) for spirometry comprises a hollow body defining a passage for an air flow, and having an inlet portion (2), for engaging the mouth of a user, an outlet portion (3) and a central portion (4) with an enlarged diameter, wherein at least one filter means (5) is placed. The filter (1) is characterized in that at least one deltaP generating member (6, 7) is placed within the central portion (4) with an enlarged diameter, said member being in series with respect to the filter means (5), configured in such a way to generate, following the passage of an air flow therethrough, a pressure differential between the two sides upstream and downstream of the member (6; 7), and in that the filter body (1) is associated with two outlets (11A, 11B) communicating, respectively, with the filter cavity (1) at the two sides of the deltaP generating member (6, 7), which can be connected to a device suitable for detecting the generated pressure differential and consequently providing a measurement of the flow rate of the air flowing through the filter (1).

## Description

### Field of the invention

The present invention relates to a disposable antimicrobial filter for spirometry, comprising a hollow body defining a passage for an air flow, and having an inlet portion, for engaging the mouth of a user, an outlet portion and a central portion with an enlarged diameter, wherein at least one filter means is placed.

Generally, antimicrobial filters of the type indicated above are used coupled to flowmeters for conducting spirometry analyzes on subjects, in order to reduce the risk of microbial cross-contamination between different subjects.

### Prior art

Spirometry is the assessment of a person's respiratory function, which can be carried out in a clinical (pulmonology), sports (for example, for evaluating fitness to practice sports) or legal (for example, in occupational medicine) context.

In particular, the evaluation tests consist of measuring the flow of exhaled/inhaled air through a flowmeter connected to the subject's mouth during particular maneuvers indicated by a specialized operator.

There are several types of flowmeters. The most common are the so-called Fleisch's pneumotachograph, Lilly's pneumotachograph, Pitot's pneumotachograph, the variable orifice flowmeter, the mass flowmeter, the turbine flowmeter and the ultrasonic flowmeter.

In the first four types of flowmeter, the flow of exhaled or inhaled air passes through a duct inside of which there is a member that generates a pressure drop, or rather, a difference in pressure between the environments upstream and downstream of this member. This pressure drop is detected by two pressure sensors (or by a single differential pressure transducer) thanks to two holes placed on the sides of the member that generates this loss.

Spirometry is an established technique in medicine. Regarding the requirements of the necessary equipment, international standardization guidelines are available, among which the following can be mentioned:
Standardization of Spirometry 2019 Update. An official American Thoracic Society and European Respiratory Society Technical Statement. Am J Respir Crit Care Med. 2019, 200(8):e70-e88;

**ERS/ATS 1997:** "Lung volume equipment and infection control"; European Respiratory Journal 1997, 10: 1928-1932.

One of the important requirements to be respected is the adoption of solutions that ensure the protection of the user's airways from contact with viruses and bacteria that may be present in the environment or in the equipment.

There are currently substantially three methods to reduce the risk of cross-contamination between different users:
1) Use of instrumentation in which all elements in contact with the exhaled and inhaled air by the user are disposable (disposable flowmeter);
2) Interposition of an antimicrobial filter between the flowmeter (reusable) and the user's mouth;
3) Use of a disposable cardboard mouthpiece and disinfection of the reusable flowmeter after each test (method almost completely abandoned).

Due to the recent COVID-19 pandemic, numerous scientific societies, including the European Respiratory Society, have recommended adopting additional precautions to protect not only the user subjected to the test, but also the surrounding environment and the health workers exposed to exhaled particles during respiratory function tests. This can be read in the following bibliographical reference:
Recommendation from ERS Group 9.1 (Respiratory function technologists/Scientists). Lung function testing during COVID-19 pandemic and beyond,
available at the following link:
   https://ers.app.box.com/s/zs1uu88wy51monr0ewd990itoz4tsn2h.

In fact, the respiratory function tests may envisage forced exhalations by the user with consequent generation of aerosol droplets, which could act as vectors of possible viruses and/or bacteria, thus increasing the risk of infecting healthcare workers present in the environment.

In view of these needs, among the methods of preventing bacterial and/or viral contamination mentioned above, the most suitable is the use of an antibacterial and/or antiviral filter.

In the standardization guidelines for the use of antibacterial and/or antiviral filters, the following requirements are required:
- the expiratory resistance of the assembly composed of the flowmeter and filter must not exceed the limit of 1.5 cm H₂O/I/sec up to flows of 14 I/sec, in order to ensure that the results are not altered;
- the connection between the filter and the flowmeter must be completely sealed, so that all the air exhaled by the user is actually measured;
- the volume of the assembly composed of the filter and flowmeter (dead space) must be as small as possible;
- the operation of the filter barrier against the passage of viruses and bacteria must be adequate and demonstrated by independent tests.

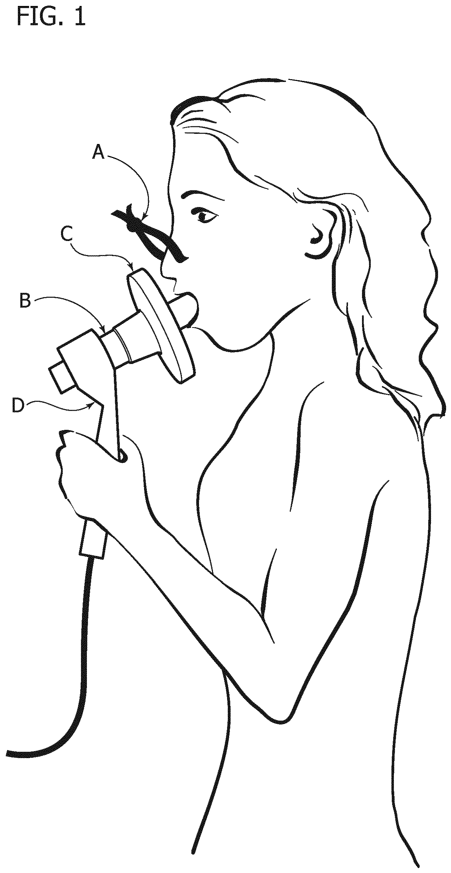

Figure 1 of the attached drawings shows the use of a flowmeter connected to an antiviral and/or antibacterial filter according to the prior art. In particular, the patient wears a nose clip A to close the nostrils, in order to convey all the exhaled air into a flowmeter B equipped with a handle D. An antibacterial and/or antiviral filter C is interposed between the flowmeter B and the patient's mouth, of the disposable type, intended to avoid contamination during the inhalation step.

Although systems similar to that shown in Figure 1 effectively function, there are some problems associated with the use of an instrument of this type that may negatively affect the reliability of the measurements and patient safety.

In particular, the response of any flowmeter may vary significantly depending on the antimicrobial filter connected thereto (the geometry of the filter affects the characteristics of the air flow, with the generation of possible turbulence).

Furthermore, the quality of the antimicrobial filters offered by the market is often poor, so there is a risk of using filters with a reduced filtering power, or that have undergone inadequate quality control processes, which do not guarantee the declared performance.

In addition to this, connection of an antimicrobial filter sometimes occurs on a flowmeter with an incompatible diameter by means of adapter fittings. This involves a probable introduction of unwanted leaks and/or an increase in the dead space of the measurement system, i.e. the volume of exhaled air that the patient is forced to breathe again, so that often the aerodynamic characteristics provided by the assembly are altered. This causes a reduction in the accuracy of the measurements made.

Finally, the use of reusable flowmeters, even if protected by antibacterial and/or antiviral filters, implies that these flowmeters must be disinfected periodically, for example, once a day, and in any case on the outer surface every time it comes into contact with a user undergoing the test.

Consequently, the need is felt for solutions without the indicated disadvantages.

### Object of the invention

One object of the present invention is to produce a disposable antimicrobial filter for spirometry of the type indicated at the beginning of the present description, wherein the risk of unwanted air leaks occurring is minimized.

An additional object of the present invention is that of producing a disposable antimicrobial filter for spirometry of the type indicated above, wherein the dead space of the measuring system is reduced.

An additional object of the present invention is that of producing a disposable antimicrobial filter for spirometry of the type indicated above, wherein the accuracy of the measurement performed is maximized.

An additional object of the present invention is to produce a disposable antimicrobial filter for spirometry of the type indicated above, capable of adequately protecting the user subjected to spirometry analysis, the surrounding environment, and the healthcare worker in charge of overseeing the analysis from microbial contamination.

### Summary of the invention

In order to achieve one or more of the aforesaid objects, the invention relates to a disposable antimicrobial filter for spirometry, comprising a hollow body defining a passage for an air flow, and having an inlet portion, for engaging the mouth of a user, an outlet portion and a central portion with an enlarged diameter, wherein at least one filter means is placed.

The filter is characterized in that at least one deltaP generating member is also placed within said central portion with an enlarged diameter, said member being in series with respect to the at least one filter means, configured in such a way as to generate, following the passage of an air flow therethrough, a pressure differential between the two sides upstream and downstream of said member, and in that two outlets communicating, respectively, with the cavity of the filter at the two sides of said at least one deltaP generating member are associated with the filter body, said outlets can be connected to a device configured to detect the aforesaid pressure differential and consequently provide a measurement of the flow rate of the air passing through the filter.

Insertion of the deltaP generating member directly into the central portion with an enlarged diameter of the disposable antimicrobial filter allows reduction of the dead space and minimizing the risk of unwanted leaks, compared to solutions wherein the deltaP generating member is included in a portion independent of the disposable antimicrobial filter. This allows the accuracy of the measurements made to be increased.

Preferably, the at least one filter means is placed between said inlet portion of the hollow body and said at least one deltaP generating member.

In one embodiment, the at least one deltaP generating member is a membrane with an orifice associated with a flexible tab, configured to deform as a result of the passage of the air flow through said orifice.

In one embodiment, the at least one deltaP generating member is an air flow resistance network.

Preferably, the disposable antimicrobial filter is a disposable antibacterial and/or antiviral filter.

Preferably, the at least one filter means comprises an electrostatic membrane.

Preferably, the central portion with an enlarged diameter is cylindrical in shape, and the at least one filter means and the at least one deltaP generating member are in the form of a circular disc.

### Detailed description of the invention

Further characteristics and advantages of the invention will become apparent from the description that follows with reference to the attached drawings, provided purely by way of non-limiting example, wherein:
- Figure 1 shows the use of an antimicrobial filter mounted on a flowmeter according to the prior art,
- Figure 2 is a perspective side view of a disposable antimicrobial filter according to the present invention,
- Figure 3 is an exploded perspective side view of a disposable antimicrobial filter according to the present invention,
- Figures 4 and 5 are cross-sectional views of embodiments of a disposable antimicrobial filter according to the present invention,
- Figures 6A and 6B are front views of details of a first embodiment of the disposable antimicrobial filter according to the present invention in two different operating conditions, and
- Figure 7 is a front view of a detail of a second embodiment of the disposable antimicrobial filter according to the present invention.

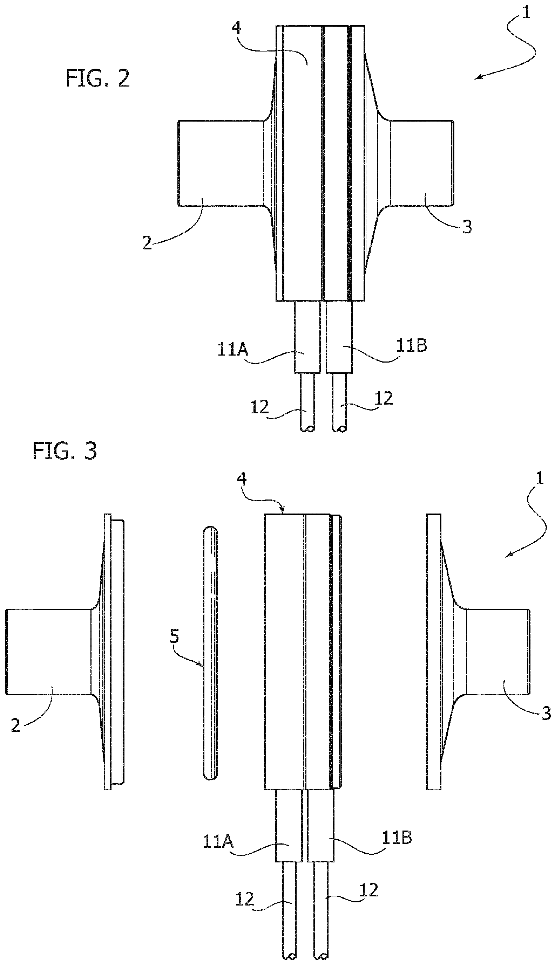

In Figure 2, numeral 1 indicates overall an antimicrobial filter that can be used to carry out spirometry analyzes in order to evaluate the respiratory function of a user. In particular, the antimicrobial filter 1 is a disposable type filter, so that it is used only once by a single user.

In the illustrated example, the antimicrobial filter 1 comprises a hollow body that defines a passage for the air flow. The hollow body comprises an inlet portion 2, for engaging the user's mouth, an outlet portion 3 and a central portion with an enlarged diameter 4.

Of course, the construction details of the hollow body of the antimicrobial filter 1 can be made in any known way. In the illustrated example, the inlet portion 2 and the outlet portion 3 are defined by two bell-shaped elements of plastic material, while the central portion with an enlarged diameter 4 is defined by two additional annular elements of plastic material 4A, 4B interlocked with each other at an annular seat 10 of the element 4B. The aforesaid elements of plastic material are rigidly connected to each other, for example, by means of adhesive and/or by welding.

In the illustrated example, the inlet portion 2, the outlet portion 3 and the central portion 4 are substantially cylindrical in shape, but it is understood that they can also be of a different shape, for example, any prismatic shape.

In the embodiment illustrated in the Figures, the inlet portion 2 and the outlet portion 3 have a substantially identical shape and size. However, it should be understood that in other embodiments, the inlet portion 2 and the outlet portion 3 have different shapes and/or dimensions.

As can be seen in Figure 3, a filter means 5 is placed inside the central portion 4, which comprises antimicrobial material, preferably antibacterial and/or antiviral.

Preferably, the filter means 5 comprises an electrostatic membrane, where "electrostatic membrane" means a membrane comprising a polymeric mixture capable of inducing the formation of a stable electric charge on the membrane itself. It should be understood that electrostatic membranes comprised in the filter means of antimicrobial filters for spirometry are known per se.

In the embodiment shown in Figure 3, the filter means 5 is in the form of a substantially circular disc having a thickness preferably between 1 and 5 millimeters. It should be understood that the filter means 5 can have a different form from that represented, for example, it can have an elliptical, square, rectangular or triangular shape In general, the filter means 5 may be in any form suitable for insertion into the central portion 4 of the hollow body of the antimicrobial filter 1.

Inside the central portion 4 there is also a deltaP generating member in series with respect to the filter means 5. Preferably, the filter means 5 is placed between the inlet portion 2 of the hollow body and the deltaP generating member.

It should be understood that the expression "deltaP generating member" as used herein refers to any member configured in such a way as to generate, following the passage of an air flow therethrough, a pressure differential between the two sides upstream and downstream of the member. An expert in the field of spirometry knows the members that fall within this definition.

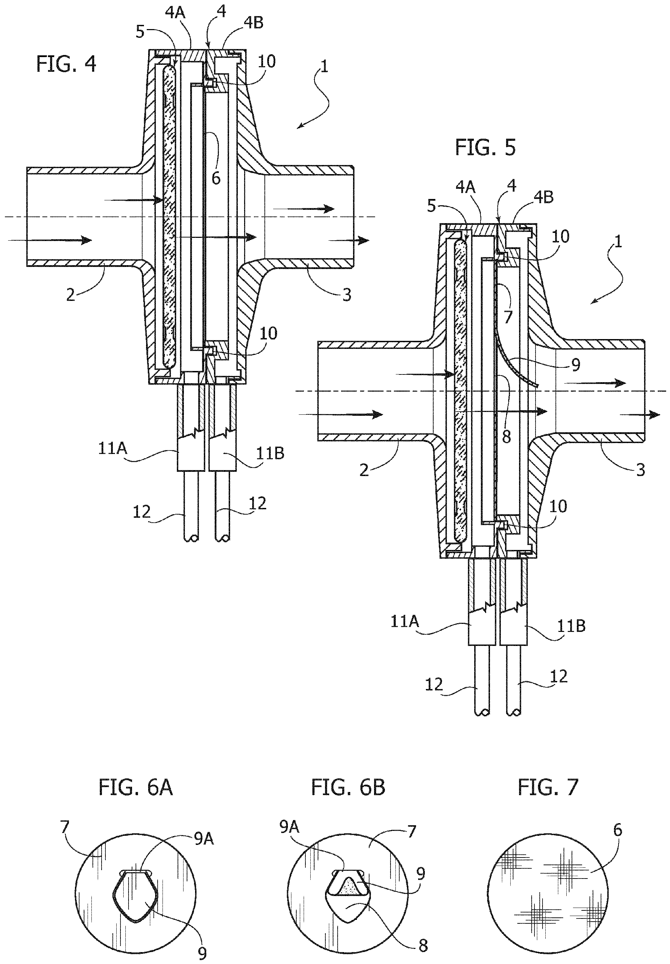

In the embodiment illustrated in Figure 4, the deltaP generating member is a network 6 of resistance to the air flow, in the form of a substantially circular disc. It should be understood that the network 6 can also have a different shape, for example elliptical, square, rectangular or triangular. In general, the network 6 may have any shape suitable for insertion into the central portion 4 of the hollow body of the antimicrobial filter 1.

Preferably, the network 6 comprises one or more materials selected from the group consisting of fabric, plastic or metal.

As can be seen in Figure 7, in the illustrated embodiment, the network 6 is formed by meshes of such size as to produce an adequate pressure drop and offer a reduced resistance to flow. It is understood that both embodiments wherein the network 6 is entirely formed by meshes, and embodiments wherein the network 6 is only partially formed by meshes fall under the scope of protection defined by the present description and, therefore, also said network includes portions in which the surface is continuous and not perforated.

In the embodiment illustrated in Figure 5, the deltaP generating member is a membrane 7 with an orifice 8 whose opening is controlled by a flexible tab 9. In particular, the flexible tab 9 is designed to be deformed by the passage of the air flow, thus passing from a first operating condition wherein it is completely extended on the orifice 8, visible in Figure 6A, to a second operating condition in which it is raised so as to leave the orifice 8, at least partially open, visible in Figure 6B.

In the embodiment illustrated in Figures 5 and 6A-6B, the membrane 7 comprises a single orifice 8. However, it should be understood that embodiments wherein the membrane 7 comprises more than a single orifice 8 also fall under the scope of protection defined by the present description.

Preferably, the membrane 7 comprises biocompatible plastic and/or steel.

In the illustrated embodiment, the membrane 7 is in the form of a substantially circular disc having a thickness preferably less than 5 millimeters. It should be understood that the membrane 7 may also have a different shape, for example, elliptical, square, rectangular or triangular. In general, the membrane 7 may have any shape suitable for insertion into the central portion 4 of the hollow body of the antimicrobial filter 1.

In the embodiment shown in Figures 6A-6B, the flexible tab 9 is associated at one end 9A with a portion of the edge of the orifice 8 formed in the membrane 7. However, it should be understood that the flexible tab 9 may also be formed in one piece with the membrane 7.

In the embodiments illustrated in the Figures, a single filter means 5 and a single deltaP generating member are inserted inside the central portion 4. However, it should be understood that embodiments wherein more than one filter means 5 and/or more than one deltaP generating member are placed within the central portion 4 of the hollow body of the antimicrobial filter 1 also fall under the scope of protection defined by the present description

In the embodiment illustrated in Figures 4 and 5, the filter means 5 is fitted into a seat of the bell-shaped element defining the inlet portion 2, while the deltaP generating member is carried by the element 4B.

As can be seen in Figures 2-5, a first outlet 11A and a second outlet 11B are also associated with the hollow body of the antimicrobial filter 1 (which in the illustrated example are associated, respectively, with the elements 4A, 4B), which respectively communicate with the cavity of the filter 1 on both sides (upstream and downstream) of the deltaP generating member. The two outlets 11A, 11B can be connected to a device configured for detecting the pressure differential generated by the deltaP generating member when an air flow passes. This device is not visible in the figures to safeguard the simplicity of the representation. The connection between the outlets 11A, 11B and the device for detecting the pressure differential can be made, for example, by means of connection tubes 12, partially illustrated in Figures 2-5.

During use of the embodiments represented in the Figures, a user whose respiratory function is to be evaluated by means of spirometry analysis places his mouth around the inlet portion 2 of the antimicrobial filter 1. On the advice of a healthcare professional, the user performs one or more inhalations and/or exhalations of air.

In the case wherein the user exhales, the exhaled air passes from the inlet portion 2 to the central portion 4, and then reaches the outlet portion 3, and is expelled into the external environment, following the path exemplified by the arrows in Figures 4-5. When the air passes through the central portion 4, it first passes through the filter means 5, and then through the deltaP generating member, thus generating the pressure differential indicative of the flow rate of the exhaled air flow. The passage through the filter means 5, which comprises antimicrobial material, prevents the leakage of microbes into the external environment, for example, bacteria and/or viruses, which are possibly exhaled by the user, thus protecting the external environment and healthcare personnel from exposure to these microbes.

Conversely, in the event that the user performs an inhalation, the air inhaled from the external environment passes into the outlet portion 3 and from there it passes into the central portion 4, and then reaches the inlet portion 2, and is thus inhaled by the user, thus following a path that is the reverse of that illustrated by the arrows in Figures 4-5. When the air passes through the central portion 4, it first passes through the deltaP generating member, thus generating the pressure differential indicative of the flow rate of the inhaled air flow, and then passes through the filter means 5. In this way, the filter means 5 prevents the user from inhaling any microbes coming from the external environment.

As can be seen in Figure 4, in the embodiment wherein the deltaP generating member is an air flow resistance network 6, the meshes of the network 6 cause a pressure drop in the air flow that passes through the hollow body and generates a pressure difference at the two sides upstream and downstream of the network 6, which is indicative of the flow rate.

As can be seen in Figure 5, in the embodiment wherein the deltaP generating member is a membrane 7 with an orifice 8 whose opening is controlled by a flexible tab 9, before the passage of the air flow the flexible tab 9 is not deformed and therefore completely covers the orifice 8, which is therefore substantially closed (Figure 6A). As the air flow passes, the flexible tab 9 is stressed and deforms, flexing proportionally to the flow rate. The deformation of the tab 9 causes an opening of the orifice 8, so that the air passes therethrough, and a pressure difference is generated between the two sides upstream and downstream of the membrane 7, due to the pressure drop to which the air flow is subjected to. As the air flow rate increases, the flexure of the tab 9 increases and, consequently, the opening level of the orifice 8 increases. This translates, therefore, into a linear relationship between the difference in pressure generated and the flow rate of the air flow that passes through the central portion 4 of the body of the filter 1.

The pressure difference generated by the deltaP generating member is detected thanks to a device connected to the filter 1 by means of two outlets 11A, 11B associated with the central portion 4 upstream and downstream of the deltaP generating member, respectively.

It is evident from the above description that the disposable antimicrobial filter subject of the present invention is extremely advantageous with respect to currently known solutions, which generally envisage the mechanical association of a filter comprising a filter means and a flowmeter in which the deltaP generating member is inserted.

In the first place, it is evident that the arrangement of the filter means and of the deltaP generating member within the central portion of the hollow body of the antimicrobial filter considerably reduces the total volume that is crossed by the air flow during the spirometry analysis. In fact, thanks to the reduction in volume, the flow of inhaled/exhaled air by the user makes a shorter path and, consequently, the risk of unwanted air leaks that could negatively affect the reliability of the measurement is considerably reduced. In addition to this, the dead space, i.e. the volume of air that remains trapped in the filter and which is consequently breathed in again by the user during the analysis, is also significantly reduced. Furthermore, the problems that occur in known solutions due to inadequate connections between filters and flowmeters are eliminated. The combination of these advantages allows greater accuracy of the spirometry analysis, which is therefore more reliable and gives a true indication of the user's respiratory function.

Secondly, the disposable antimicrobial filter subject of the present invention has a higher efficiency than the known solutions in preventing the risk of a microbial cross-contamination between the user and the healthcare personnel who supervise the spirometry analysis.

In particular, insertion of the deltaP generating member inside the central portion of the hollow body of the antimicrobial filter avoids the need to use a flowmeter outside the filter and, consequently, the healthcare staff are not forced to manipulate and disinfect the flowmeter between the analyzes of different users. Consequently, having to handle fewer instruments reduces the risk of healthcare personnel coming into contact with possible viruses and/or bacteria of the user, and speeds up the time required for preparing the subsequent spirometry analysis on the subsequent patient.

In addition to this, studies and investigations conducted by the Applicant have shown that the expiratory resistance of the disposable antimicrobial filter subject of the present invention respects the limits set by the international recommendations (American Thoracic Society, European Respiratory Society).

Furthermore, insertion of the deltaP generating member directly into the central portion of the hollow body of the filter avoids the onset of problems of variation of the response that occur in known flowmeters due to the connection with a distinct body that acts as an antimicrobial filter.

Of course, without prejudice to the principle of the invention, the details of construction and the embodiments may vary widely with respect to those described and illustrated purely by way of example, without departing from the scope of protection of the present invention, as defined by the attached claims.

## Claims

1. A disposable antimicrobial filter (1) for spirometry, comprising a hollow body defining a passage for an air flow, and having an inlet portion (2), for engaging the mouth of a user, an outlet portion (3) and a central portion (4) with an enlarged diameter, wherein at least one filter means (5) is placed.
said filter (1) being **characterized in that** within said central portion (4) with an enlarged diameter at least one deltaP generating member (6; 7) is also placed, in series with respect to the at least one filter means (5), configured in such a way as to generate, following the passage of an air flow therethrough, a pressure differential between the two sides upstream and downstream of said member (6; 7), and
**in that** two outlets (11A; 11B) are associated with the filter body (1), communicating, respectively, with the cavity of the filter (1) at the two sides of said at least one deltaP generating member (6; 7), which can be connected to a device configured to detect the aforesaid pressure differential and consequently provides a measurement of the flow rate of the air flow passing through the filter (1).

2. A disposable antimicrobial filter according to claim 1, **characterized in that** said at least one filter means (5) is placed between said inlet portion (2) of the hollow body and said at least one deltaP generating member (6; 7).

3. A disposable antimicrobial filter according to claim 1, **characterized in that** said at least one deltaP generating member (6; 7) is a membrane (7) with an orifice (8) with which a flexible tab (9) is associated, configured to deform following the passage of the air flow through said orifice.

4. A disposable antimicrobial filter according to claim 1, **characterized in that** said at least one deltaP generating member (6; 7) is an air flow resistance network (6).

5. A disposable antimicrobial filter according to claim 1, **characterized in that** said at least one filter means (5) comprises an electrostatic membrane.

6. A disposable antimicrobial filter according to any of the preceding claims, **characterized in that** the inlet portion (2) and the outlet portion (3) are defined by two bell-shaped elements of plastic material, while the central portion with an enlarged diameter (4) is defined by two additional annular elements of plastic material (4A, 4B), interposed between said bell-shaped elements, and
**in that** said filter means (5) is fitted into a seat of the bell-shaped element defining the inlet portion (2), while the deltaP generating member (6; 7) is carried by one of said additional elements of plastic material (4B)
